# EUROPÄISCHE PATENTSCHRIFT

(11) **EP 0 579 159 B1**
(45) Veröffentlichungstag und Bekanntmachung des Hinweises auf die Patenterteilung: **14.01.1998**
(21) Anmeldenummer: 93111165.2
(22) Anmeldetag: 13.07.1993
(51) Int. Cl.: C07C 69/33, C07C 67/08, B01F 17/34, A61K 7/48

(54) **Verfahren zur Herstellung von Polyglycerinfettsäureestergemischen, und die Verwendung in kosmetischen, pharmazeutischen und/oder chemisch-technischen Zubereitungen**
Method for preparing mixtures of polyglycerol fatty acid esters and their application in cosmetic, pharmaceutical and chemical-technical compositions
Procédé de préparation de mélanges à base d'esters polyglycéroliques d'acides gras et leurs utilisations dans des préparations cosmétiques, pharmaceutiques et chimico-techniques

(30) Priorität: 16.07.1992 DE 4223407
(43) Veröffentlichungstag der Anmeldung: 19.01.1994
(73) Patentinhaber: Solvay Alkali GmbH, 30173 Hannover (DE)
(72) Erfinder: Jakobson, Gerald, Dr., D-4134 Rheinberg 2 (DE); Siemanowski, Werner, Dr., D-4134 Rheinberg 1 (DE); Uhlig, Karl-Heinz, D-4150 Krefeld (DE)
(74) Vertreter: Lauer, Dieter, Dr.

(56) Entgegenhaltungen:
- EP-A- 0 451 461
- EP-A- 0 499 700
- US-A- 5 045 337
- DATABASE WPI Week 9226, Derwent Publications Ltd., London, GB; AN 92-214261 & JP-A-4 145 046 (NEW JAPAN CHEM CO LTD) 19. Mai 1992
- DATABASE WPI Week 9120, Derwent Publications Ltd., London, GB; AN 91-144825 & JP-A-3 081 252 (LION CORP) 5. April 1991

## Beschreibung

Die vorliegende Erfindung betrifft ein Verfahren zur Herstellung von Polyglycerinfettsäureestergemischen durch partielle Veresterung eines Polyglyceringemisches aus Monoglycerin, Diglycerin, Triglycerin, Tetraglycerin und höheren Polyglycerinen mit mindestens einer gesättigten Fettsäure mit 8 bis 18 C-Atomen, wobei die Fettsäure oder das Fettsäuregemisch einen Anteil von 0 bis 15 gew% an ungesättigten Fettsäuren aufweist, in Gegenwart eines sauren Katalysators.

Die Erfindung betrifft weiterhin Verdikkungsmittel und O/W-Emulgatoren mit ganz bestimmter Zusammensetzung hinsichtlich des eingesetzten Polyglycerin fettsäureestergemisches sowie die Verwendung der erfindungsgemäß hergestellten Polyglycerinfettsäureestergemische.

Die hauptsächliche industrielle Darstellung von Polyglycerinfettsäureestergemischen basiert auf der direkten Veresterung von Fettsäuren mit Polyglycerinen verschiedener Kettenlänge (die als Polyglyceringemisch oder einfach als Polyglycerin bezeichnet werden) in Gegenwart eines alkalischen Katalysators bei erhöhter Temperatur. Die bekannten Verfahren sind jedoch nicht selektiv, so daß man ein Gemisch nichtdefinierter Ester von Polyglycerinen mit gesättigten und ungesättigten Fettsäuren erhält. Die Herstellung von Polyglycerinfettsäureestern definierter Zusammensetzung erfordert eine komplizierte Reaktionsführung (im allgemeinen ist eine Einführung von Schutzgruppen in die Polyglycerinverbindungen erforderlich), und die Reaktionsbedingungen sind schwierig zu kontrollieren.

Aus DE-OS 40 05 819 bzw. EP 451 461 ist bereits die Verwendung von Mischungen aus Polyglycerinfettsäureestern als Emulgatoren in kosmetischen und pharmazeutischen Zubereitungen bekannt, wobei das Polyglycerinfettsäureestergemisch durch partielle Veresterung von Polyglycerinmischungen aus 0 bis 5 Gew.-% Monoglycerin, 15 bis 40 Gew.-% Diglycerin, 30 bis 55 Gew.-% Triglycerin, 10 bis 25 Gew.-% Tetraglycerin und 0 bis 30 Gew.-% höheren Polyglycerinen mit mindestens einer ungesättigten Fettsäure mit 16 bis 22 C-Atomen hergestellt wird. Die Veresterung der Polyglycerine erfolgt zwar partiell, jedoch wird nur ein nichtdefiniertes Gemisch von Polyglycerinfettsäureestern mit einem allgemeinen Veresterungsgrad zwischen 20 und 70 % erhalten.

Ein Verfahren, wie es Gegenstand der vorliegenden Erfindung ist und das die Herstellung von Polyglycerinfettsäureestergemischen mit ganz bestimmmter Zusammensetzung und Verteilung der Ester in dem Gemisch erlaubt, ist diesen Veröffentlichungen nicht zu entnehmen.

Der DE 400 5 819 bzw. der ihr entsprechenden EP 451 461 kann man entnehmen, daß diese Polyglycerinfettsäureester mit einem Veresterungsgrad von 20-70% geeignet sind lagerstabile Emulsionen herzustellen. Angaben, ob das verwendete Estergemisch kosmetischen Zubereitungen pflegende Eigenschaften verleiht, kann diesen Schriften nicht entnommen werden.

Weiterhin ist aus der DE-OS 39 02 274 ein Wasch-, Reinigungs- und/oder Körperreinigungsmittel bekannt, das neben einem ionogenen und/oder amphoteren Tensid Monofettsäureester mit C₈ bis C₁₈ des Diglycerins und/oder Difettsäureester mit C₈ bis C₁₈ des Tetraglycerins enthält. Dieses Wasch-, Reinigungs- und/oder Körperreinigungsmittel zeichnet sich durch eine gute Verarbeitbarkeit und Hautverträglichkeit aus und ist biologisch leicht abbaubar. Es hat jedoch den Nachteil, daß es in einem relativ teuren und arbeitsaufwendigen Mehrstufenprozeß hergestellt werden muß und normalerweise als festes Produkt anfällt. Bei der Weiterverarbeitung des Produktes, z.B. in Zubereitungen usw., treten verarbeitungstechnische Schwierigkeiten auf, so z.B. daß das Produkt nicht bei Raumtemperatur gepumpt werden kann. Es besteht das Risiko der Verstopfung von Zuleitungs- und Dosiervorrichtungen. Zur Auflösung des Produktes sind entweder Wärmezufuhr und/oder starke Scherkräfte erforderlich.

In der europäischen Patentanmeldung EP-A 0 286 636 ist eine emulgierte oder solubilisierte Sterinzusammensetzung offenbart, wobei die Sterine in einer wäßrigen Lösung einer Polyhydroxyverbindung oder in einer flüssigen Polyhydroxyverbindung, die Saccharose- und/oder Polyglycerinfettsäureester enthält, emulgiert oder solubilisiert sind. Die als Lösungsvermittler und Emulgatoren wirkenden Polyglycerinfettsäureester werden durch Veresterung von Polyglycerinen mit einem Polymerisationsgrad von 4 bis 10 und gesättigten und/oder ungesättigten Fettsäuren mit 10 bis 24 C-Atomen erhalten, wobei der Gehalt an Monoestern wenigstens 50 Gew.-% beträgt.

Auch diese Patentanmeldung enthält keine Hinweise auf ein Verfahren und ein danach hergestelltes Polyglycerinfettsäureestergemisch, wie es Gegenstand der vorliegenden Erfindung ist.

Aufgabe der vorliegenden Erfindung war es, ein Verfahren zur Herstellung von Polyglycerinfettsäureestergemischen definierter Zusammensetzung bereitzustellen, das eine einfache und kostengünstige Reaktionsführung erlaubt und eine gleichbleibende Qualität und reproduzierbare Zusammensetzung des Endproduktes gewährleistet.

Weiterhin sollte das nach dem erfindungsgemäßen Verfahren hergstellte Polyglycerinfettsäureestergemisch bei Raumtemperatur flüssig sein, so daß es leicht verarbeitbar oder einarbeitbar ist. Es sollte außerdem eine verdickende, rückfettende und/oder hautpflegende Wirkung aufweisen sowie als Emulgator und/oder Solubilisierungsmittel in einem weiten Anwendungsbereich, der u. a. kosmetische, pharmazeutische und chemisch-technische Produkte umfaßt, einsetzbar sein, wobei eine ökologische und toxikologische Unbedenklichkeit ebenfalls gewährleistet sein muß.

Erfindungsgemäß wurde festgestellt, daß dieser Aufgabe ein Verfahren gerecht wird, das dadurch gekennzeichnet ist, daß das Polyglycerin, das (bezogen auf 100 Gew.-Teile Polyglycerin) aus

| | |
|---|---|
| 0,1 bis 20 Gew.-% | Monoglycerin, |
| 20 bis 35 Gew.-% | Diglycerin, |
| 30 bis 40 Gew.-% | Triglycerin und |
| 49,9 bis 5 Gew.-% | Tetra- und höheren Polyglycerinen |

besteht, mit einer oder mehreren Fettsäuren, ausgewählt aus den gesättigten C₈- bis C₁₈-Fettsäuren, wobei die Fettsäure oder das Fettsäuregemisch einen Anteil 0 bis 15 Gew.-% an ungesättigten Fettsäuren aufweist, in einem Mol-Verhältnis des Polyglycerins zu der Fettsäure oder dem Fettsäuregemisch von

| | |
|---|---|
| 0,3 : 1,5 bis 1,5 : 0,5, | vorzugsweise |
| 0,5 : 1 bis 1,2 : 1, | |

in Gegenwart mindestens eines Katalysators, vorzugsweise eines sauren Katalysators, und bei Unterdruck umgesetzt wird, indem das Reaktionsgemisch zunächst bis auf 140°C, vorzugsweise 145°C, aufgeheizt und der Druck bis auf 600 mbar, vorzugsweise 500 mbar, reduziert wird und dann das Reaktionsgemisch in einem Temperaturbereich

| | |
|---|---|
| 140 bis 220°C, | vorzugsweise |
| 145 bis 190°C, | |

stufenweise oder kontinuierlich, über ein Temperaturprogramm gesteuert, erhitzt wird und dabei der Druck stufenweise oder kontinuierlich, über ein Druckprogramm gesteuert,

| | |
|---|---|
| von 600 auf 10 mbar, | vorzugsweise |
| von 500 auf 20 mbar, | |

erniedrigt wird, wobei das entstehende Reaktionswasser kontinuierlich abdestilliert und bei Erreichen einer Säurezahl von < 3 das erhaltene Polyglycerinfettsäureestergemisch abgekühlt und gegebenenfalls für besondere Anwendungen aufgearbeitet und/oder gereinigt wird.

Nach einer bevorzugten Ausführungsform des erfindungsgemäßen Verfahrens wird ein Polyglyceringemisch eingesetzt, das (bezogen auf 100 Gew.-teile Polyglycerin) aus

| | |
|---|---|
| 6 bis 16 Gew.-% | Monoglycerin, |
| 23 bis 33 Gew.-% | Diglycerin, |
| 31 bis 37 Gew.-% | Triglycerin und |
| 40 bis 14 Gew.-% | Tetra- und höheren Polyglycerinen |

besteht oder diese Bestandteile enthält.

Überraschend wurde bei diesem Verfahren gefunden, daß der Zusatz von Glycerin in den beanspruchten Mengen eine Verbesserung der Löslichkeit des entsprechenden Polyglycerinfettsäureestergemisches in wäßrigen Formulierungen bewirkt.

Nach einer bevorzugten Ausführungsform des erfindungsgemäßen Verfahrens erfolgt eine Reinigung und/oder Aufarbeitung des hergestellten Polyglycerinfettsäureestergemisches, um nicht umgesetzte Polyglycerinanteile aus dem Verfahrensprodukt zu entfernen, was für bestimmte Anwendungen von Vorteil oder notwendig ist.

Hierzu wird das Polyglycerinfettsäureestergemisch auf 30 bis 110°C, vorzugsweise 60 bis 80°C, abgekühlt und nachfolgend einer Extraktionsbehandlung unterworfen, indem das Polyglycerinfettsäureestergemisch mit einem organisch-chemischen Lösungsmittel oder Lösemittelgemisch versetzt und anschließend mit Wasser in mindestens einer Extraktionsstufe extrahiert wird, wobei in der ersten Extraktionsstufe zusätzlich eine der Säurezahl des Polyglycerinfettsäureestergemisches entsprechende und/oder der Katalysatormenge mindestens äquivalente Gewichtsmenge einer anorganischen und/oder organischen, basisch reagierenden Verbindung zugesetzt wird, und die nach der Extraktion verbleibende organische Phase von dem eingesetzten organischen Lösungsmittel und dem Restwassergehalt durch Destillation, vorzugsweise Vakuumdestillation oder Vakuumverdampfung, befreit wird.

Das in der Extraktionsbehandlung eingesetzte organisch-chemische Lösungsmittel oder Lösemittelgemisch weist vorzugsweise ein Wasseraufnahmevermögen von weniger als 30 Gew.-%, vorzugsweise weniger als 20 Gew.-% (bezogen auf 100 Gew.-Teile des organisch-chemischen Lösemittels oder Lösemittelgemisches), auf und/oder bildet mit Wasser bei der Destillation oder in der Gasphase ein azeotropes Gemisch.

Ein organisch-chemisches Lösemittel, das diese Eigenschaften aufweist, ist Ethylacetat, das vorzugsweise als mit Wasser gesättigte organische Phase in dem Extraktionsverfahren eingesetzt wird.

Weitere, erfindungsgemäß verwendbare organisch-chemische Lösemittel sind u. a. Butanol und/oder Toluol.

Die Extraktion wird nach einer bevorzugten Ausführungsform in mehr als zwei Stufen, vorzugsweise drei Stufen, durchgeführt.

Die Neutralisation des Katalysators wird erfindungsgemäß vorzugsweise mit Natriumhydroxid, insbesondere mit wäßriger Natriumhydroxidlösung durchgeführt, wobei die alkalische Verbindung oder Lösung in der ersten Extraktionsstufe dem organisch-chemischen Lösemittel oder Lösemittelgemisch und/oder der Wasserphase zugegeben wird.

Als weitere Neutralisationsmittel sind Alkalicarbonate, vorzugsweise Natriumcarbonat und/oder Kaliumcarbonat, und/oder ein basischer Ionenaustauscher geeignet, die jeweils in der ersten Extraktionsstufe dem organisch-chemischen Lösemittel oder Lösemittelgemisch und/oder der Wasserphase zugegeben werden.

Läßt man das erfindungsgemäß hergestellte Polyglycerinfettsäureestergemisch vor einer Weiterverarbeitung und/oder Reinigung mehr als 0,5, vorzugsweise 1 bis 10 Stunden, stehen, so können aus dem Produktgemisch nicht umgesetzte Anteile an Polyglycerin gegebenenfalls ausgeschieden und abgetrennt werden.

Die gleichbleibende Qualität des Endproduktes, die definierte Zusammensetzung des Estergemisches und die Reproduzierbarkeit des Reaktionsergebnisses werden bei diesem Verfahren insbesondere durch die kontrollierte Reaktionsführung hinsichtlich Aufheizung und gleichzeitiger Druckerniedrigung in dem Reaktionsgemisch sowie durch die exakte Einhaltung bestimmter Druck- und Temperaturbereiche erhalten.

Die Reaktionsführung des erfindungsgemäßen Verfahrens kann kontinuierlich, über ein entsprechendes Temperatur- und Druckprogramm gesteuert, erfolgen, oder diskontinuierlich durch stufenweise Erwärmung und Druckverminderung.

Dabei wurde gefunden, die Aufheizung des Reaktionsgemisches im Temperaturbereich von 140 bis 220°C, vorzugsweise 145 bis 190°C, und die Druckerniedrigung von 600 auf 10 mbar, vorzugsweise von 500 auf 20 mbar, in einem Zeitintervall von

| | |
|---|---|
| 2 bis 6 Stunden, | vorzugsweise |
| 3 bis 4 Stunden, | |

durchzuführen.

Gemäß einer weiteren bevorzugten Ausführungsform des erfindungsgemäßen Verfahrens erfolgt die Aufheizung des Reaktionsgemisches im Temperaturbereich von 140 bis 220°C, vorzugsweise 145 bis 190°C, stufenweise über

| | |
|---|---|
| 3 bis 6 Stufen, | vorzugsweise |
| 4 bis 5 Stufen. | |

Damit korrespondierend kann die Erniedrigung des Druckes von 600 auf 10 mbar, vorzugsweise 500 auf 20 mbar, schrittweise in einer bestimmten Anzahl von Stufen vorgenommen werden, vorzugsweise über

| | |
|---|---|
| 3 bis 6 Stufen, | insbesondere |
| 4 bis 5 Stufen. | |

Die Veresterungsreaktion nach dem erfindungsgemäßen Verfahren wird in Gegenwart eines Katalysators durchgeführt, vorzugsweise in Gegenwart einer sauren, sulfonsäuregruppenhaltigen Verbindung, wie beispielsweise Dodecylbenzolsufonsäure oder andere Alkylbenzolsulfonsäuren.

Nach einer Ausführungsform wird dieser saure Katalysator in Kombination mit einer zweiten sauren, katalytisch und reduzierend wirkenden Verbindung eingesetzt. Eine in dieser Hinsicht geeignete Verbindung ist hypophosphorige Säure.

Gemäß einer weiteren vorteilhaften Ausführungsform des erfindungsgemäßen Verfahrens wird das Reaktionsgemisch in Gegenwart eines inerten Gases, vorzugsweise Stickstoff, umgesetzt.

Die vorliegende Erfindung betrifft weiterhin flüssige Verdickungsmittel mit hautpflegender Wirkung und O/W-Emulgatoren mit hautpflegenden Eigenschaften für Cremes oder Salben, unter Verwendung des erfindungsgemäß hergestellten Polyglycerin fettsäureestergemisches.

Das Polyglycerinfettsäureestergemisch weist (bezogen auf 100 Gewichtsteile des Polyglycerinfettsäureestergemisches)
(A) einen Anteil von

| | |
|---|---|
| 0,1 bis 20 Gew.-% | Monoglycerinfettsäureester, |
| 20 bis 35 Gew.-% | Diglycerinfettsäureester, |
| 30 bis 40 Gew.-% | Triglycerinfettsäureester und |
| 49,9 bis 5 Gew.-% | Tetra- und höheren Polyglycerinfettsäureestern, |

wobei die Fettsäurekomponente aus einer oder mehreren Fettsäuren, ausgewählt aus den gesättigten C₈- bis C₁₈-Fettsäuren mit einen Anteil von 0 bis 15 Gew.-% an ungesättigten Fettsäuren, besteht, und
(B) einen Veresterungsgrad von

| | |
|---|---|
| 20 bis 80 Gew.-% | Fettsäuremonoestern und |
| 80 bis 20 Gew.-% | Fettsäurediestern, mit einem Anteil von 0 bis 15 Gew.-% Tri- und höheren Estern, |

auf, wobei keine oder nur geringe Mengen (weniger als 5 Gew.-%) an freiem Polyglycerin in dem Polyglycerinfettsäureestergemisch enthalten sind.

Nach einer vorzugsweisen Ausführungsform weist das Polyglycerinfettsäureestergemisch (bezogen auf 100 Gewichtsteile des Polyglycerinfettsäureestergemisches)
(A) einen Anteil von

| | |
|---|---|
| 6 bis 16 Gew.-% | Monoglycerinfettsäureester, |
| 23 bis 33 Gew.-% | Diglycerinfettsäureester, |
| 31 bis 37 Gew.-% | Triglycerinfettsäureester und |
| 40 bis 14 Gew.-% | Tetra- und höheren Polyglycerinfettsäureestern, |

wobei die Fettsäurekomponente aus einer oder mehreren Fettsäuren, ausgewählt aus den gesättigten C₈- bis C₁₈-Fettsäuren mit einen Anteil von 0 bis 10 Gew.-% an ungesättigten Fettsäuren, besteht, und
(B) einen Veresterungsgrad von

| | |
|---|---|
| 30 bis 70 Gew.-% | Fettsäuremonoestern und |
| 70 bis 30 Gew.-% | Fettsäurediestern, mit einem Anteil von 0,5 bis 7 Gew.-% Tri- und höheren Estern, |

auf, wobei keine oder nur geringe Mengen (weniger als 2,5 Gew.-%) an freiem Polyglycerin in dem Polyglycerinfettsäureestergemisch enthalten sind.

Das erfindungsgemäße flüssige Verdickungsmittel mit hautpflegender Wirkung, besteht aus oder enthält ein Polyglycerinfettsäureestergemisch, das (bezogen auf 100 Gewichtsteile des Polyglycerinfettsaureestergemisches)
(A) einen Anteil von

| | |
|---|---|
| 0,1 bis 20 Gew.-% | Monoglycerinfettsäureester, |
| 20 bis 35 Gew.-% | Diglycerinfettsäureester, |
| 30 bis 40 Gew.-% | Triglycerinfettsäureester und |
| 49,9 bis 5 Gew.-% | Tetra- und höheren Polyglycerinfettsäureestern, |

wobei die Fettsäurekomponente aus einer oder mehreren Fettsäuren, ausgewählt aus den gesättigten C₈- bis C₁₈-Fettsäuren, vorzugsweise Caprinsäure, besteht, und
(B) einen Veresterungsgrad von

| | |
|---|---|
| 20 bis 80 Gew.-% | Fettsäuremonoestern und |
| 80 bis 20 Gew.-% | Fettsäurediestern, mit einem Anteil von 0 bis 15 Gew.-% Tri- und höheren Estern, |

aufweist, wobei keine oder nur geringe Mengen (weniger als 5 Gew.-%) an freiem Polyglycerin in dem Polyglycerinfettsäureestergemisch enthalten sind.

Ein derartiges Verdickungsmittel weist nicht nur hautpflegende Wirkung auf, sondern es ist außerdem kalt verarbeitbar und läßt sich aufgrund seines bei Raumtemperatur flüssigen Zustandes leicht dosieren und damit in kosmetische oder pharmazeutische Formulierungen auf einfache Weise einarbeiten. Die überraschenden Wirkungen des Verdickungsmittels sind dabei insbesondere auf die Verwendung von Caprinsäure als Fettsäurekomponente zurückzuführen, wobei diese Fettsäure in reiner Form ohne oder nur mit Spurenanteilen an ungesättigten Fettsäuren eingesetzt wird.

Nach einer bevorzugten Ausführungsform besteht das flüssige Verdickungsmittel mit hautpflegender Wirkung aus einem Polyglycerinfettsäureestergemisch, das (bezogen auf 100 Gewichtsteile des Polyglycerinfettsäureestergemisches)
(A) einen Anteil von

| | |
|---|---|
| 6 bis 16 Gew.-% | Monoglycerinfettsäureester, |
| 23 bis 33 Gew.-% | Diglycerinfettsäureester, |

| | |
|---|---|
| 31 bis 37 Gew.-% | Triglycerinfettsäureester und |
| 40 bis 14 Gew.-% | Tetra- und höheren Polyglycerinfettäureestern, |

wobei die Fettsäurekomponente aus einer oder mehreren Fettsäuren, ausgewählt aus den gesättigten C₈- bis C₁₈-Fettsäuren, vorzugsweise Caprinsäure, besteht, und
(B) einen Veresterungsgrad von

| | |
|---|---|
| 30 bis 70 Gew.-% | Fettsäuremonoestern und |
| 70 bis 30 Gew.-% | Fettsäurediestern, mit einem Anteil von 0,5 bis 7 Gew.-% Tri- und höheren Estern, |

aufweist, wobei keine oder nur geringe Mengen (weniger als 2,5 Gew.-%) an freiem Polyglycerin in dem Polyglycerinfettsäureestergeinisch enthalten sind.

Der erfindungsgemäße O/W-Emulgator mit hautpflegenden Eigenschaften zur Verwendung in Cremes oder Salben, besteht aus oder enthält ein Polyglycerinfettsäureestergemisch, das (bezogen auf 100 Gewichtsteile des Polyglycerinfettsäureestergemisches)
(A) einen Anteil von

| | |
|---|---|
| 0,1 bis 20 Gew.-% | Monoglycerinfettsäureester, |
| 20 bis 35 Gew.-% | Diglycerinfettsäureester, |
| 30 bis 40 Gew.-% | Triglycerinfettsäureester und |
| 49,9 bis 5 Gew.-% | Tetra- und höheren Polyglycerinfettsäureestern, |

wobei die Fettsäurekomponente aus einer oder mehreren Fettsäuren, ausgewählt aus den gesättigten C₈- bis C₁₈-Fettsäuren mit einen Anteil von 0 bis 15 Gew.-% an ungesättigten Fettsäuren, vorzugsweise Cocosfettsäure, besteht, und
(B) einen Veresterungsgrad von

| | |
|---|---|
| 20 bis 80 Gew.-% | Fettsäuremonoestern und |
| 80 bis 20 Gew.-% | Fettsäurediestern, mit einem Anteil von 0 bis 15 Gew.-% Tri- und höheren Estern, |

aufweist, wobei keine oder nur geringe Mengen (weniger als 5 Gew.-%) an freiem Polyglycerin in dem Polyglycerinfettsäureestergemisch enthalten sind.

Der erfindungsgemäße O/W-Emulgator ist ausgezeichnet für Cremes und Salben geeignet, da er hautpflegende Eigenschaften aufweist und in topischen Formulierungen ein sehr angenehmes Hautgefühl bewirkt. Die eingesetzte Cocosfettsäure ist ein Gemisch aus gesättigten und ungesättigten Fettsäuren, wobei der Anteil an ungesättigten Fettsäuren zwischen 0 und 15 Gew.-% betragen kann.

Nach einer bevorzugten Ausführungsform besteht der O/W-Emulgator mit hautpflegenden Eigenschaften zur Verwendung in Cremes oder Salben aus einem Polyglycerinfettsäureestergemisch, das (bezogen auf 100 Gewichtsteile des Polyglycerinfettsäureestergemisches)
(A) einen Anteil von

| | |
|---|---|
| 6 bis 16 Gew.-% | Monoglycerinfettsäureester, |
| 23 bis 33 Gew.-% | Diglycerinfettsäureester, |
| 31 bis 37 Gew.-% | Triglycerinfettsäureester und |
| 40 bis 14 Gew.-% | Tetra- und höheren Polyglycerinfettfsäureestern, |

wobei die Fettsäurekomponente aus einer oder mehreren Fettsäuren, ausgewählt aus den gesättigten C₈- bis C₁₈-Fettsäuren mit einen Anteil von 4 bis 10 Gew.-% an ungesättigten Fettsäuren, vorzugsweise Cocosfettsäure, besteht, und
(B) einen Veresterungsgrad von

| | |
|---|---|
| 30 bis 70 Gew.-% | Fettsäuremonoestern und |
| 70 bis 30 Gew.-% | Fettsäurediestern, mit einem Anteil von 0,5 bis 7 Gew.-% Tri- und höheren Estern, |

aufweist, wobei keine oder nur geringe Mengen (weniger als 2,5 Gew.-%) an freiem Polyglycerin in dem Polyglycerinfettsäureestergemisch enthalten sind.

Die Erfindung betrifft weiterhin die Verwendung des nach dem erfindungsgemäßen Verfahren hergestellten Polyglycerinfettsäureestergemisches als Solubilisierungsmittel, Verdickungsmittel, Rückfetter und/oder Emulgator in kosmetischen, pharmazeutischen oder chemisch-technischen Zubereitungen.

Das erfindungsgemäß hergestellte Polyglycerinfettsäureestergemisch ist vielseitig verwendbar. Vorzugsweise dient es zur Solubilisierung und/oder Emulgierung von ätherischen Ölen und Parfümölen. Es eignet sich daher ausgezeichnet zur Herstellung von Badezusätzen, insbesondere Badeölen oder ölbädern.

Des weiteren wird das erfindungsgemäß hergestellte Polyglycerinfettsäureestergemisch als Hautpflegeadditiv und/oder Wasch-, Reinigungs- oder Körperreinigungsmittel, Duschgel oder Duschmittel, Schaumbademittel, flüssiges Handreinigungsmittel oder Haarshampoo verwendet, da es gleichzeitig auch oberflächenaktive Eigenschaften besitzt und neben milder Reinigungswirkung bestimmte zusätzliche Eigenschaften, wie z.B. einen Rückfettungseffekt und ein angenehmes Hautgefühl während und nach dem Reinigungsvorgang, ein verbessertes Fließverhalten und darüber hinaus eine dermatologische und toxikologische Unbedenklichkeit aufweist.

Weitere Einsatzbereiche für das erfindungsgemäß hergestellte Polyglycerinfettsäureestergemisch sind Lebensmittel sowie Arzneimittel, Salben, pharmazeutische und kosmetische Zubereitungen jeglicher Art, wo das Polyglycerinfettsäureestergemisch als Solubilisierungs- und/oder Verdickungsmittel und/oder Emulgator wirkt. Seine vielseitige Verwendbarkeit ergibt sich u.a. dadurch, daß das Polyglycerinfettsäureestergemisch einerseits leicht verarbeitbar und andererseits haut- und körperneutral bzw. -pflegend ist. Kosmetische Zubereitungen unter Verwendung dieses Polyglycerinfettsäureestergemisches Vermitteln ein optimales pflegendes Gefühl. Bei der Anwendung auf der Haut, z.B. in kosmetischen Zubereitungen, Hautdesinfektionsmitteln, Salben, Einreibungen und dergleichen hat das Polyglycerinfettsäureestergemisch eine rückfettende Wirkung.

Es war nicht zu erwarten, daß diese Eigenschaften und Ergebnisse mit dem erfindungsgemäß hergestellten Polyglycerinfettsäureestergemisch erreicht werden konnten und daß zudem auch solche Anforderungen, wie die der dermatologischen Wirksamkeit sowie der toxikologischen Unbedenklichkeit, erfüllt werden.

Des weiteren eignet sich das Polyglycerinfettsäureestergemisch vorzüglich für technische Anwendungen, vorzugsweise als Emulgator in Bohrölen oder Bohrflüssigkeiten sowie Schmierölen, sowie als Netz-und/oder Dispergiermittel in technischen Reinigungsmitteln oder als Emulgator und/oder Dispergiermittel in Farbzubereitungen, vorzugsweise in Dispersionsfarben, oder Bautenschutzmitteln, insbesondere Holzschutzfarben und -lasuren.

In anwendungsfertigen Formulierungen kann das Polyglycerinfettsäureestergemisch mit weiteren Zusatz- oder Hilfsmitteln problemlos kombiniert werden. In Wasch-, Reinigungs- und/oder Körperreinigungsmittel können bevorzugt zusätzlich Elektrolyte und/oder weitere Tenside sowie Lösungs- und/oder Verdünnungsmittel eingesetzt werden. Des weiteren kann das Polyglycerinfettsäureestergenisch zusammen mit Konservierungs-, Parfümierungs-, Färbemitteln, pharmazeutischen Wirkstoffen, Stellmitteln zur pH-Regulierung, Komplexierungsmitteln zur Maskierung von Metallionen, Hautpflegemitteln und/oder Verdickungsmitteln und anderen Verbindungen wie Kolloiden, desinfizierenden Wirkstoffen, fungiziden, insektiziden oder antibakteriellen Wirkstoffen, Korrosionsschutzmitteln usw. verwendet werden.

Die nachfolgenden Ausführungsbeispiele sollen die Erfindung erläutern, ohne sie darauf zu beschränken.

### 1. Ausführungsbeispiel

### Herstellung von Polyglycerincocoat

In einem 2-Liter-Vierhalskolben, der mit Rührwerk, Wasserabscheider, Thermometer und Gaseinleitungsrohr versehen ist, wird eine Mischung von 977 g = 4,16 mol Polyglycerin (bestehend aus ca. 15 % Glycerin, ca. 25 % Diglycerin, ca. 31 % Triglycerin, ca. 17 % Tetraglycerin und ca. 12 % höheren Oligomeren), 878 g = 4,16 mol Cocosfettsäure, 2,8 g Dodecylbenzolsulfonsäure und 1,1 g hypophosphorige Säure unter Rühren und gleichzeitigem Durchleiten von Inertgas während ca. 3 h bei 500 bis 20 mbar von 145°C bis max. 165°C erhitzt, wobei das Reaktionswasser kontinuierlich destillativ entfernt wird. Bei Erreichen einer Säurezahl von < 2 wird das Reaktionsgemisch auf ca. 65 bis 70°C heruntergekühlt und nicht umgesetzte Anteile an Polyglycerin wie folgt entfernt:
Das Reaktionsgemisch wird in Ethylacetat (halber Volumenanteil, mit Wasser gesättigt) gelöst und dreistufig mit Wasser extrahiert. In der 1. Stufe wird dem wäßrigen Ethylacetat die der Säurezahl entsprechende Menge an Natronlauge zugesetzt. Die Gesamtmenge des zur Extraktion verwendeten Wassers beträgt ca. 87 Vol.-% des eingesetzten Esters. Die Summe der extrahierten Anteile an Polyglycerin nach Abdestillation der Lösungsmittel beträgt ca. 611 g. Die nach der Extraktion verbleibende organische Phase wird im Vakuum eingedampft.

| **Kennzahlen** | **Anteile der Ester in Gew.-%** |
|---|---|
| Säurezahl: 2,5 mg KOH/g | Monoester: ca. 25 |
| Verseifungszahl: 167,0 mg KOH/g | Diester: ca. 65 |
| Viskosität bei 50°C: 450 mPa.s | höhere Ester: ca. 10 |

### 2. Ausführungsbeispiel

### Herstellung von Polyglycerincaprinat

In einem 2-Liter-Vierhalskolben, der mit Rührwerk, Wasserabscheider, Thermometer und Gaseinleitungsrohr versehen ist, wird eine Mischung von 939 g = 4 mol Polyglycerin (bestehend aus ca. 15 % Glycerin, ca. 25 % Diglycerin, ca. 31 % Triglycerin, ca. 17 % Tetraglycerin und ca. 12 % höheren Oligomeren), 688 g = 4 mol Caprinsäure, 2,8 g Dodecylbenzolsulfonsäure und 1,1 g hypophosphorige Säure unter Rühren und gleichzeitigem Durchleiten von Inertgas während ca. 3 h bei 500 bis 20 mbar von 145°C bis max. 165°C erhitzt, wobei das Reaktionswasser kontinuierlich destillativ entfernt wird. Bei Erreichen einer Säurezahl von < 2 wird das Reaktionsgemisch auf ca. 65 bis 70°C heruntergekühlt und nicht umgesetzte Anteile an Polyglycerin wie folgt entfernt:
Das Reaktionsgemisch wird in Ethylacetat (halber Volumenanteil, mit Wasser gesättigt) gelöst und dreistufig mit Wasser extrahiert. In der 1. Stufe wird dem wäßrigen Ethylacetat die der Säurezahl entsprechende Menge an Natronlauge zugesetzt. Die Gesamtmenge des zur Extraktion verwendeten Wassers beträgt ca. 87 Vol.-% des eingesetzten Esters. Die Summe der extrahierten Anteile an Polyglycerin nach Abdestillation der Lösungsmittel beträgt 309 g. Die nach der Extraktion verbleibende organische Phase wird im Vakuum eingedampft.

| **Kennzahlen** | **Anteile der Ester in Gew.-%** |
|---|---|
| Säurezahl: 1,6 mg KOH/g | Monoester: ca. 50 |
| Verseifungszahl: 174,8 mg KOH/g | Diester: ca. 40 |
| Viskosität bei 20°C: 4600 mPas.s | höhere Ester: ca. 10 |

### 3. Ausführungsbeispiel

### Herstellung von Polyglycerinlaurat

In einem 2-Liter-Vierhalskolben, der mit Rührwerk, Wasserabscheider, Thermometer und Gaseinleitungsrohr versehen ist, wird eine Mischung von 858 g = 3,65 mol Polyglycerin (bestehend aus ca. 15 % Glycerin, ca. 25 % Diglycerin, ca. 31 % Triglycerin, ca. 17 % Tetraglycerin und ca. 12 % höheren Oligomeren), 811 g = 4,06 mol Laurinsäure, 2,8 g Dodecylbenzolsulfonsäure und 1,1 g hypophosphorige Säure unter Rühren und gleichzeitigem Durchleiten von Inertgas während ca. 3 h bei 500 bis 20 mbar von 145°C bis max. 165 °C erhitzt, wobei das Reaktionswasser kontinuierlich destillativ entfernt wird. Bei Erreichen einer Säurezahl von < 2 wird das Reaktionsgemisch auf ca. 60 bis 65°C heruntergekühlt und nicht umgesetzte Anteile an Polyglycerin wie folgt entfernt:
Das Reaktionsgemisch wird in Ethylacetat (halber Volumenanteil, mit Wasser gesättigt) gelöst und dreistufig mit Wasser extrahiert. In der 1. Stufe wird dem wäßrigen Ethylacetat die der Säurezahl entsprechende Menge an Natronlauge zugesetzt. Die Gesamtmenge des zur Extraktion verwendeten Wassers beträgt ca. 87 Vol.-% des eingesetzten Esters. Die Summe der extrahierten Anteile an Polyglycerin nach Abdestillation der Lösungsmittel beträgt ca. 253 g. Die nach der Extraktion verbleibende organische Phase wird im Vakuum eingedampft.

| **Kennzahlen** | **Anteile der Ester in Gew.-%** |
|---|---|
| Säurezahl: 1,1 mg KOH/g | Monoester: ca. 35 |
| Verseifungszahl: 166,3 mg KOH/g | Diester: ca. 55 |
| Viskosität bei 20°C: 10100 mPa.s | höhere Ester: ca. 10 |

### 4. Ausführungsbeispiel

### Herstellung von Polyglycerinisostearat

In einem 2-Liter-Vierhalskolben, der mit Rührwerk, Wasserabscheider, Thermometer und Gaseinleitungsrohr versehen ist, wird eine Mischung von 470 g = 2,0 mol Polyglycerin (bestehend aus ca. 15 % Glycerin, ca. 25 % Diglycerin, ca. 31 % Triglycerin, ca. 17 % Tetraglycerin und ca. 12 % höheren Oligomeren), 1080 g = 3,8 mol Isostearinsäure, 2,8 g Dodecylbenzolsulfonsäure und 1,1 g hypophosphorige Säure unter Rühren und gleichzeitigem Durchleiten von Inertgas während ca. 4 h bei 500 bis 20 mbar von 145°C bis max. 170°C erhitzt, wobei das Reaktionswasser kontinuierlich destillativ entfernt wird. Bei Erreichen einer Säurezahl von < 2 wird das Reaktionsgemisch auf ca. 60 bis 65°C heruntergekühlt und nicht umgesetzte Anteile an Polyglycerin wie folgt entfernt:
Das Reaktionsgemisch wird in Ethylacetat (halber Volumenanteil, mit Wasser gesättigt) gelöst und einstufig mit Wasser extrahiert. In der 1. Stufe wird dem wäßrigen Ethylacetat die der Säurezahl entsprechende Menge an Natronlauge zugesetzt. Die Gesamtmenge des zur Extraktion verwendeten Wassers beträgt ca. 50 Vol.-% des eingesetzten Esters. Die Summe der extrahierten Anteile an Polyglycerin nach Abdestillation der Lösungsmittel beträgt ca. 67 g. Die nach der Extraktion verbleibende organische Phase wird im Vakuum eingedampft.

| **Kennzahlen** | |
|---|---|
| Säurezahl | 1,5 mg KOH/g |
| Verseifungszahl | 153,6 mg KOH/g |
| Viskosität bei 25°C | 5500 mPa.s |

### 5. Ausführungsbeispiel

### Herstellung von Polyglycerinisostearat

In einem 2-Liter-Vierhalskolben, der mit Rührwerk, Wasserabscheider, Thermometer und Gaseinleitungsrohr versehen ist, wird eine Mischung von 352 g = 1,5 mol Polyglycerin (bestehend aus ca. 15 % Glycerin, ca. 25 % Diglycerin, ca. 31 Triglycerin, ca. 17 % Tetraglycerin und ca. 12 % höheren Oligomeren), 1108 g = 3,9 mol Isostearinsäure, 2,8 g Dodecylbenzolsulfonsäure und 1,1 g hypophosphorige Säure unter Rühren und gleichzeitigem Durchleiten von Inertgas während ca. 4 h bei 500 bis 20 mbar von 145°C bis max. 190°C erhitzt, wobei das Reaktionswasser kontinuierlich destillativ entfernt wird. Bei Erreichen einer Säurezahl von < 2 wird das Reaktionsgemisch auf ca. 60 bis 65°C heruntergekühlt; nicht umgesetzte Anteile an Polyglycerin werden nicht entfernt.

| **Kennzahlen** | |
|---|---|
| Säurezahl | 2,2 mg KOH/g |
| Verseifungszahl | 157,4 mg KOH/g |
| Viskosität bei 25°C | 2200 mPa.s |

### Anwendungsbeispiele für das erfindungsgemäß hergestellte Polyglycerinfettsäureestergemisch

### 1. O/W Hautcreme mit dem erfindungsgem. PGLC-Cocoat als O/W-Emulgator

| | |
|---|---|
| 1,5 % | Polyglycerincocoat (hergestellt gemäß Ausführungsbeispiel 1) |
| 2,0 % | Cetyl-stearylalkohol |
| 9,1 % | Glycerinmono/distearat |
| 4,0 % | Cetearyl Isononanoate (Cetial SN, Henkel) |
| 5,0 % | Paraffinöl DAB, dickflüssig |
| 1,6 % | Calendulaextrakt |
| 8,0 % | Diglycerin |
| 0,12 % | Polyacrylsäure (Carbopol 940, BF Goodrich) |
| 0,22 % | Neutralisationsmittel |
| 0,05 % | Konservierungsmittel |
| 0,3 % | Parfüm |
| 68,11 % | Wasser vollentsalzt |

Das Mittel ist eine schwachfettende, glatte, softige Creme mit sehr raschem Einziehvermögen und sehr gutem Hautgefühl.

Stabilität bei 20°C und 40°C: > 2 Monate

### 2. Standard-Haarschampoo mit erf.-gem. PGLC-Caprinat als Verdicker und Pflegeadditiv

Gesamttensidgehalt: 17 %

| | |
|---|---|
| 50 % | Na-laurylethersulfat 28 %ig |
| 3 % | Polyglycerincaprinat (hergstellt gemäß Ausführungsbeispiel 2) |
| 2 % | Natriumchlorid |
| 0,05 % | Konservierungsmittel |
| 0,2 % | Parfüm |
| 44,75 % | Wasser vollentsalzt |

Viskosität: 4000 mPa.s

| | |
|---|---|
| Schaumzahl nach Ross/Miles, DIN 53902 | |
| 1 g/l WAS, 40 °C, | dest. Wasser: |
| nach 30 sec | 200 mm |
| nach 3 min | 195 mm |
| nach 5 min | 195 mm |

### 3. Hautmildes Körperreinigungsmittel und hautmildes Haarshampoo mit erf.-gem. PGLC-Caprinat als Verdicker und Pflegeadditiv

Gesamttensidgehalt: 12 %

| | |
|---|---|
| 21,8 % | Na-laurylethersulfat 28 %ig |
| 2,0 % | Polyglycerincaprinat (hergestellt gemäß Ausführungsbeispiel 2) |
| 6,7 % | Alkylamidobetain 30 % |
| 5,0 % | Di-Na-fettalkoholpolyglykolethersulfosuccinat 40 %ig |
| 1,7 % | Natriumchlorid |
| 0,05 % | Konservierungsmittel |
| 0,3 % | Parfüm |
| 62,45 % | Wasser vollentsalzt |

Viskosität: 4100 m Pa.s

| | |
|---|---|
| Schaumzahl nach Ross/Miles, DIN 53902 | |
| 1g/l WAS, 40 °C, | dest. Wasser: |
| nach 30 sec | 195 mm |
| nach 3 min | 190 mm |
| nach 5 min | 185 mm |

### 4. O/W Körperlotion mit erf.-gem. PGLC-Laurat

| | |
|---|---|
| 3,5 % | Polyglycerinlaurat (hergestellt gemäß Ausführungsbeispiel 3) |
| 7,0 % | Capryl-Caprinsäure-Triglycerid (Neutralöl) |
| 3,0 % | Propylenglykol-dicaprylat-dicaprinat (Miglyol 840, Hüls AG) |
| 5,0 % | Weizenkeimöl |
| 0,21 % | Polyacrylsäure (Carbopol 940, BF Goodrich) |
| 0,32 % | Neutralisationsmittel |
| 0,05 % | Konservierungsmittel |
| 0,6 % | Parfüm |
| 80,32 % | Wasser vollentsalzt |

Mittelfettende, viskose Lotion mit sehr raschem Einziehvermögen und sehr angenehmem Hautgefühl.

Stabilität bei 20°C und 40°C: > 2 Monate

### 5. W/O Hautcreme mit erf.-gem. PGLC-Isostearat als W/O Emulgator

Molverhältnis: 1 mol PGLC : 2,5 mol Isostearinsäure

| | |
|---|---|
| 2,5 % | Polyglycerinisostearat (hergestellt gemäß Ausführungsbeispiel 5) |
| 3,5 % | Decyloleat (Cetiol V, Henkel) |
| 3,0 % | Dioctylcyclohexan (Cetiol S, Henkel) |
| 3,5 % | Paraffinöl DAB, dickflüssig |
| 0,7 % | Magnesiumstearat |
| 0,3 % | Aluminiumstearat |
| 4,0 % | Diglycerin |
| 0,3 % | Magnesiumheptahydrat |
| 0,05 % | Konservierungsmittel |
| 0,3 % | Parfüm |
| 81,85 % | Wasser vollentsalzt |

Schwachfettende, weiße, glänzende, softige Creme mit einem für W/O-Cremes ungewöhnlich raschem Einziehungsvermögen auf der Haut und einem hervorragenden Hautgefühl.

Stabilität bei 20°C und 40°C: > 2 Monate

### 6. (pflegendes) ölbad

| | |
|---|---|
| 43 % | Polyglycerincaprinat (hergestellt gemäß Ausführungsbeispiel 2) |
| 42 % | Rosmarinöl |
| 15 % | Wasser vollentsalzt |

## Patentansprüche

1. Verfahren zur Herstellung von Polyglycerinfettsäureestergemischen durch partielle Veresterung eines Polyglyceringemisches aus Mono-, Di-, Tri-, Tetra- und höheren Polyglycerinen mit mindestens einer gesättigten Fettsäure mit C₈ bis C₁₈ C-Atomen, wobei die Fettsäure oder das Fettsäuregemisch einen Anteil von 0 bis 15 Gew.-% an ungesättigten Fettsäuren aufweist, in Gegenwart eines sauren Katalysators, dadurch gekennzeichnet, daß ein Polyglyceringemisch aus (bezogen auf 100 Gew.-Teile Polyglycerin)
| | |
|---|---|
| 0,1 bis 20 Gew.-% | Monoglycerin |
| 20 bis 35 Gew.-% | Diglycerin |
| 30 bis 40 Gew.-% | Triglycerin und |
| 5 bis 49,9 Gew.-% | Tetra- und höheren Polyglycerinen |
mit der Fettsäure oder dem Fettsäuregemisch in einem Molverhältnis von Polyglycerin zu Fettsäure oder Fettsäuregemisch von 0,3:1,5 bis 1,5:0,5 zu einem Polyglycerinfettsäureestergemisch aus (bezogen auf 100 Gew.-Teile Polyglycerinfettsäureestergemisch)
| | |
|---|---|
| 0,1 bis 20 Gew.-% | Monoglycerinfettsäureester |
| 20 bis 35 Gew.-% | Diglycerinfettsäureester |
| 30 bis 40 Gew.-% | Triglycerinfettsäureester und |
| 49,9 bis 5 Gew.-% | Tetra- und höhere Polyglycerinfettsäureester |
mit einem Veresterungsgrad von
| | |
|---|---|
| 20 bis 80 Gew.-% | Fettsäuremonoester und |
| 80 bis 20 Gew.-% | Fettsäurediester, mit einem Anteil von |
| 0 bis 15 Gew.-% | Tri- und höheren Estern |
umgesetzt werden, indem das Reaktionsgemisch zunächst bis auf 140 °C aufgeheizt und der Druck bis auf 600 mbar reduziert wird und danach das Reaktionsgemisch in einem Temperaturbereich von 140 bis 220 °C stufenweise oder kontinuierlich, über ein Temperaturprogramm gesteuert, erhitzt und der Druck stufenweise oder kontinuierlich, über ein Druckprogramm gesteuert, bis auf 10 mbar reduziert wird, wobei das entstehende Reaktionswasser kontinuierlich abdestilliert und bei Erreichen einer Säurezahl von <3 das erhaltene Polyglyceringemisch abgekühlt und gegebenenfalls aufgearbeitet und/oder gereinigt wird.

2. Verfahren nach Anspruch 1, dadurch gekennzeichnet, daß ein Polyglyceringemisch aus (bezogen auf 100 Gew.-Teile Polyglycerin)
| | |
|---|---|
| 6 bis 16 Gew.-% | Monoglycerin |
| 23 bis 33 Gew.-% | Diglycerin |
| 31 bis 37 Gew.-% | Triglycerin und |
| 14 bis 40 Gew.-% | Tetra- und höhere Polyglycerine |
mit der Fettsäurekomponente zu einem Polyglycerinfettsäureestergemisch aus
| | |
|---|---|
| 6 bis 16 Gew.-% | Monoglycerinfettsäureester |
| 23 bis 33 Gew.-% | Diglycerinfettsäureester |
| 31 bis 37 Gew.-% | Triglycerinfettsäurester und |
| 14 bis 40 Gew.-% | Tetra- und höheren Polyglycerinfettsäureestern |
mit einem Veresterungsgrad von
| | |
|---|---|
| 30 bis 70 Gew.-% | Fettsäuremonoester und |
| 70 bis 30 Gew.-% | Fettsäurediester, mit einem Anteil von |
| 0,5 bis 10 Gew.-% | Tri- und höheren Estern |
umgesetzt werden.

3. Verfahren nach den Ansprüchen 1 oder 2, dadurch gekennzeichnet, daß das erhaltene Polyglycerinfettsäureestergemisch auf 30 bis 110°C, abgekühlt und nachfolgend von nicht umgesetzten Polyglycerinanteilen durch Extraktion befreit wird, indem das Polyglycerinfettsäureestergemisch mit einem organisch-chemischen Lösungsmittel oder Lösemittelgemisch versetzt und anschließend mit Wasser in mindestens einer Extraktionsstufe extrahiert wird, wobei in der ersten Extraktionsstufe zusatzlich eine der Säurezahl des Polyglycerinfettsäureestergemisches entsprechende und/oder der Katalysatormenge mindestens äquivalente Gewichtsmenge einer anorganischen und/oder organischen, basisch reagierenden Verbindung zugesetzt wird, und die nach der Extraktion verbleibende organische Phase von dem eingesetzten organischen Lösungsmittel und dem Restwassergehalt durch Destillation, vorzugsweise Vakuumdestillation oder Vakuumverdampfung, befreit wird.

4. Verfahren nach einem oder mehreren der Ansprüche 1 bis 3, dadurch gekennzeichnet, daß das organisch-chemische Lösemittel oder Lösemittelgemisch ein Wasseraufnahmevermögen von weniger als 30 Gew.-%, (bezogen auf 100 Gew.-Teile des organisch-chemischen Lösemittels oder Lösemittelgemisches), aufweist und/oder mit Wasser bei der Destillation oder in der Gasphase ein azeotropes Gemisch bildet.

5. Verfahren nach einem oder mehreren der Ansprüche 1 bis 4, dadurch gekennzeichnet, daß als organisch-chemisches Lösemittel Ethylacetat, eingesetzt wird.

6. Verfahren nach einem oder mehreren der Ansprüche 1 bis 5, dadurch gekennzeichnet, daß die Extraktion in mehr als zwei Stufen, durchgeführt wird.

7. Verfahren nach einem oder mehreren der Ansprüche 1 bis 6, dadurch gekennzeichnet, daß zur Neutralisation des Katalysators Natriumhydroxid, in der ersten Extraktionsstufe dem organisch-chemischen Lösemittel oder Lösemittelgemisch und/oder der Wasserphase zugegeben wird.

8. Verfahren nach einem oder mehreren der Ansprüche 1 bis 7, dadurch gekennzeichnet, daß zur Neutralisation des Katalysators Alkalicarbonate, Natriumcarbonat und/oder Kaliumcarbonat, und/oder ein basischer Ionenaustauscher, in der ersten Extraktionsstufe dem organisch-chemischen Lösemittel oder Lösemittelgemisch und/oder der Wasserphase zugegeben werden.

9. Verfahren nach einem oder mehreren der Ansprüche 1 bis 8, dadurch gekennzeichnet, daß das erhaltene Polyglycerinfetcsäureestergemisch vor einer Weiterverarbeitung und/oder Reinigung mehr als 0,5 h, stehengelassen wird und gegebenenfalls ausgeschiedene Anteile an Polyglycerinen abgetrennt erden.

10. Verfahren nach einem oder mehreren der Ansprüche 1 bis 9, dadurch gekennzeichnet, daß die Aufheizung des Reaktionsgemisches im Temperaturbereich von 140 bis 220°C, und die Druckerniedrigung von 600 auf 10 mbar, in einem Zeitintervall von
2 bis 6 Stunden,
erfolgen.

11. Verfahren nach einem oder mehreren der Ansprüche 1 bis 10, dadurch gekennzeichnet, daß die Aufheizung des Reaktionsgemisches im Temperaturbereich von 140 bis 220°C, stufenweise über
3 bis 6 Stufen,
erfolgt.

12. Verfahren nach einem oder mehreren der Ansprüche 1 bis 11, dadurch gekennzeichnet, daß die Druckerniedrigung von 600 auf 10 mbar, stufenweise über
3 bis 6 Stufen,
erfolgt.

13. Verfahren nach einem oder mehreren der Ansprüche 1 bis 12, dadurch gekennzeichnet, daß der saure Katalysator, in Kombination mit einer sauren, reduzierend wirkenden Verbindung, eingesetzt wird.

14. Verfahren nach einem oder mehreren der Ansprüche 1 bis 13, dadurch gekennzeichnet, daß das Reaktionsgemisch in Gegenwart eines inerten Gases, umgesetzt wird.

15. Verfahren nach Anspruch 1 bis 14, dadurch gekennzeichnet, daß das Molverhältnis Polyglycerin zu Fettsäure oder Fettsäuregemisch 0,5:1 bis 1,2:1 beträgt und zur Umsetzung das Reaktionsgemisch zunächst auf 145 °C aufgeheizt und der Druck auf 500 mbar reduziert wird und danach das Reaktionsgemisch auf 190 °C erhitzt und der Druck auf 20 mbar reduziert wird.

16. Flüssiges Verdickungsmittel mit hautpflegender Wirkung, hergestellt nach dem Verfahren gemäß einem oder mehreren der Ansprüche 1 bis 14, dadurch gekennzeichnet, daß das Polyglycerinfettsäureestergemisch (bezogen auf 100 Gewichtsteile des Polyglycerinfettsäuregemisches)
(A) einen Anteil von
| | |
|---|---|
| 0,1 bis 20 Gew.-% | Monoglycerinfettsäureester, |
| 20 bis 35 Gew.-% | Diglycerinfettsäureester, |
| 30 bis 40 Gew.-% | Triglycerinfettsäureester und |
| 49,9 bis 5 Gew.-% | Tetra- und höheren Polyglycerinfettsäureestern, |
wobei die Fettsäurekomponente aus einer oder mehreren Fettsäuren, ausgewählt aus den gesättigten C₈- bis C₁₈-Fettsäuren, besteht, und
(B) einen Veresterungsgrad von
| | |
|---|---|
| 20 bis 80 Gew.-% | Fettsäuremonoestern und |
| 80 bis 20 Gew.-% | Fettsäurediestern, mit einem Anteil von 0 bis 15 Gew.-% Tri- und höheren Estern, |
aufweist, wobei keine oder nur geringe Mengen (weniger als 5 Gew.-%) an freiem Polyglycerin in dem Polyglycerinfettsäureestergemisch enthalten sind.

17. Flüssiges Verdickungsmittel mit hautpflegender Wirkung nach Anspruch 16, dadurch gekennzeichnet, daß das Polyglycerinfettsäureestergemisch (bezogen auf 100 Gewichtsteile des Polyglycerinfettsäureestergemisches)
(A) einen Anteil von
| | |
|---|---|
| 6 bis 16 Gew.-% | Monoglycerinfettsäureester, |
| 23 bis 33 Gew.-% | Diglycerinfettsäureester, |
| 31 bis 37 Gew.-% | Triglycerinfettsäureeter und |
| 40 bis 14 Gew.-% | Tetra- und höheren Polyglycerinfettsäureestern, |
wobei die Fettsäurekomponente aus einer oder mehreren Fettsäuren, ausgewählt aus den gesättigten C₈- bis C₁₈-Fettsäuren, besteht, und
(B) einen Veresterungsgrad von
| | |
|---|---|
| 30 bis 70 Gew.-% | Fettsäuremonoestern und |
| 70 bis 30 Gew.-% | Fettsäurediestern, mit einem Anteil von 0,5 bis 10 Gew.-% Tri- und höheren Estern, |
aufweist, wobei keine oder nur geringe Mengen (weniger als 2,5 Gew.-%) an freiem Polyglycerin in dem Polyglycerinfettsäureestergemisch enthalten sind.

18. O/W-Emulgator mit hautpflegenden Eigenschaften zur Verwendung in Cremes oder Salben, hergestellt nach dem Verfahren gemäß einem oder mehreren der Ansprüche 1 bis 14, dadurch gekennzeichnet, daß das Polyglycerinfettsäureestergemisch (bezogen auf 100 Gewichtsteile des Polyglycerinfettsäureestergemisches)
(A) einen Anteil von
| | |
|---|---|
| 0,1 bis 20 Gew.-% | Monoglycerinfettsäureester, |
| 20 bis 35 Gew.-% | Diglycerinfettsäureester, |
| 30 bis 40 Gew.-% | Triglycerinfettsäureester und |
| 49,9 bis 5 Gew.-% | Tetra- und höheren Polyglycerinfettsäureestern, |
wobei die Fettsäurekomponente aus einer oder mehreren Fettsäuren, ausgewählt aus den gesattigten C₈- bis C₁₈-Fettsäuren mit einen Anteil von 0 bis 15 Gew.-% an ungesättigten Fettsäuren, besteht, und
(B) einen Veresterungsgrad von
| | |
|---|---|
| 20 bis 80 Gew.-% | Fettsäuremonoestern und |
| 80 bis 20 Gew.-% | Fettsäurediestern, mit einem Anteil von 0 bis 15 Gew.-% Tri- und höheren Estern, |
aufweist, wobei keine oder nur geringe Mengen (weniger als 5 Gew.-%) an freiem Polyglycerin in dem Polyglycerinfettsäureestergenisch enthalten sind.

19. O/W-Emulgator mit hautpflegenden Eigenschaften zur Verwendung in Cremes oder Salben nach Anspruch 18, dadurch gekennzeichnet, daß das Polyglycerinfettsäureestergemisch (bezogen auf 100 Gewichtsteile des Polyglycerinfettsäureestergemisches)
(A) einen Anteil von
| | |
|---|---|
| 6 bis 16 Gew.-% | Monoglycerinfettsäureester, |
| 23 bis 33 Gew.-% | Diglycerinfettsäureester, |
| 31 bis 37 Gew.-% | Triglycerinfettsäureeter und |
| 40 bis 14 Gew.-% | Tetra- und höheren Polyglycerinfettsäureestern, |
wobei die Fettsäurekomponente aus einer oder mehreren Fettsäuren, ausgewählt aus den gesättigten C₈- bis C₁₈-Fettsäuren mit einen Anteil von 4 bis 10 Gew.-% an ungesättigten Fettsäuren, besteht, und
(B) einen Veresterungsgrad von
| | |
|---|---|
| 30 bis 70 Gew.-% | Fettsäuremonoestern und |
| 70 bis 30 Gew.-% | Fettsäurediestern mit einem Anteil von 0,5 bis 10 Gew.-% Tri- und höheren Estern |
aufweist, wobei keine oder nur geringe Mengen (weniger als 2,5 Gew.-%) an freiem Polyglycerin in dem Polyglycerinfettsäureescergemisch enthalten sind.

20. Verwendung des Polyglycerinfettsäureestergemisches, hergestellt nach dem Verfahren gemäß einem oder mehreren der Ansprüche 1 bis 14, als Solubilislerungsmittel, Verdickungsmittel, Rückfetter und/oder Emulgator in kosmetischen, pharmazeutischen oder chemisch-technischen Zubereitungen.

21. Verwendung des Polyglycerinfettsäureestergemisches nach Anspruch 20 zur Solubilisierung und/oder Emulgierung von ätherischen ölen und Parfümölen.

22. Verwendung des Polyglycerinfettsäureestergemisches nach Anspruch 20 als Hautpflegeadditiv und/oder Wasch-, Reinigungs- oder Körperreinigungsmittel.

23. Verwendung des Polyglycerinfettsäureestergemisches nach Anspruch 20 als Emulgator in Bohrölen oder Bohrflüssigkeiten.

24. Verwendung des Polyglycerinfettsäureestergemisches nach Anspruch 20 als Netz- und/oder Dispergiermittel in technischen Reinigungsmitteln.

25. Verwendung des Polyglycerinfettsäureestergemisches nach Anspruch 20 als Emulgator und/oder Dispergiermittel in Farbzubereitungen.

## Claims

1. Process for the preparation of polyglycerol fatty acid ester mixtures by partial esterification of a polyglycerol mixture consisting of monoglycerol, diglycerol, triglycerol, tetraglycerol and higher polyglycerols with at least one saturated fatty acid with C₈ to C₁₈ C atoms, the fatty acid or the fatty acid mixture having a content of 0 to 15% by weight unsaturated fatty acids, in the presence of an acidic catalyst, characterised in that a polyglycerol mixture of (relative to 100 parts by weight of polyglycerol)
| | |
|---|---|
| 0.1 to 20 % | by weight of monoglycerol, |
| 20 to 35 % | by weight of diglycerol, |
| 30 to 40 % | by weight of triglycerol and |
| 5 to 49.9 % | by weight of tetra- and higher polyglycerols, |
is reacted with the fatty acid or the fatty acid mixture in a molar ratio of polyglycerol to the fatty acid or fatty acid mixture of 0.3 : 1.5 to 1.5 : 0.5,
to give a polyglycerol fatty acid ester mixture of (relative to 100 parts by weight of polyglycerol fatty acid mixture)
| | |
|---|---|
| 0.1 to 20 % | by weight of monoglycerol fatty acid ester, |
| 20 to 35 % | by weight of diglycerol fatty acid ester, |
| 30 to 40 % | by weight of triglycerol fatty acid ester and |
| 49.9 to 5 % | by weight of tetra- and higher polyglycerol fatty acid esters, |
with a degree of esterification of
| | |
|---|---|
| 20 to 80 % | by weight of fatty acid monoesters and |
| 80 to 20 % | by weight of fatty acid diesters, having a content of 0 to 15 % by weight of tri- and higher esters, |
by initially heating the reaction mixture to 140°C and reducing the pressure to 600 mbar and then heating the reaction mixture in a temperature range from 140 to 220°C, stepwise or continuously, controlled by means of a temperature program, and reducing the pressure, stepwise or continuously, controlled by means of a pressure program, to 10 mbar, the resultant water of reaction being continuously removed by distillation and on attaining an acid number of < 3, the resulting polyglycerol mixture being cooled and optionally worked up and/or purified.

2. Process according to Claim 1, characterised in that a polyglycerol mixture of (relative to 100 parts by weight of polyglycerol)
| | |
|---|---|
| 6 to 16 % | by weight of monoglycerol, |
| 23 to 33 % | by weight of diglycerol, |
| 31 to 37 % | by weight of triglycerol and |
| 14 to 40 % | by weight of tetra- and higher polyglycerols |
is reacted with the fatty acid component to give a polyglycerol fatty acid ester mixture of
| | |
|---|---|
| 6 to 16 % | by weight of monoglycerol fatty acid ester, |
| 23 to 33 % | by weight of diglycerol fatty acid ester, |
| 31 to 37 % | by weight of triglycerol fatty acid ester and |
| 14 to 40 % | by weight of tetra- and higher polyglycerol fatty acid esters, |
with a degree of esterification of
| | |
|---|---|
| 30 to 70 % | by weight of fatty acid monoesters and |
| 70 to 30 % | by weight of fatty acid diesters, having a content of 0.5 to 10 % by weight of tri- and higher esters. |

3. Process according to Claims 1 or 2,
characterised in that the polyglycerol fatty acid ester mixture obtained is cooled to 30 to 110°C, and subsequently freed from unreacted polyglycerol contents by extraction, by adding to the polyglycerol fatty acid ester mixture an organic chemical solvent or solvent mixture and then extracting with water in at least one extraction step, an amount by weight of an inorganic and/or organic, basic compound corresponding to the acid number of the polyglycerol fatty acid ester mixture and/or at least equivalent to the amount of catalyst additionally being added in the first extraction step, and the organic phase remaining after the extraction being freed from the organic solvent employed and the residual water content by distillation, preferably vacuum distillation or vacuum evaporation.

4. Process according to one or more of Claims 1 to 3, characterised in that the organic chemical solvent or solvent mixture has a water absorption capacity of less than 30 % by weight (relative to 100 parts by weight of the organic chemical solvent or solvent mixture), and/or forms an azeotropic mixture with water during the distillation or in the gas phase.

5. Process according to one or more of Claims 1 to 4, characterised in that the organic chemical solvent employed is ethyl acetate.

6. Process according to one or more of Claims 1 to 5, characterised in that the extraction is carried out in more than two steps.

7. Process according to one or more of Claims 1 to 6, characterised in that sodium hydroxide is added to the organic chemical solvent or solvent mixture and/or the water phase in the first extraction step to neutralise the catalyst.

8. Process according to one or more of Claims 1 to 7, characterised in that alkali metal carbonates, sodium carbonate and/or potassium carbonate, and/or a basic ion exchanger are added to the organic chemical solvent or solvent mixture and/or the water phase in the first extraction step to neutralise the catalyst.

9. Process according to one or more of Claims 1 to 8, characterised in that the polyglycerol fatty acid ester mixture obtained is allowed to stand for more than 0.5 h before further processing and/or purification and optionally precipitated contents of polyglycerols are separated off.

10. Process according to one or more of Claims 1 to 9, characterised in that the heating of the reaction mixture in the temperature range from 140 to 220°C and the pressure reduction from 600 to 10 mbar are carried out in a time interval of
2 to 6 hours.

11. Process according to one or more of Claims 1 to 10, characterised in that the heating of the reaction mixture in the temperature range from 140 to 220°C is carried out stepwise over
3 to 6 steps.

12. Process according to one or more of Claims 1 to 11, characterised in that the pressure reduction from 600 to 10 mbar is carried out stepwise over
3 to 6 steps.

13. Process according to one or more of Claims 1 to 12, characterised in that the acidic catalyst is employed in combination with an acidic, reducing compound.

14. Process according to one or more of Claims 1 to 13, characterised in that the reaction mixture is reacted in the presence of an inert gas.

15. Process according to Claims 1 to 14,
characterised in that the molar ratio of polyglycerol to fatty acid or fatty acid mixture is 0.5:1 to 1.2:1, and for reaction the reaction mixture is first heated to 145°C and the pressure is reduced to 500 mbar, and then the reaction mixture is heated to 190°C and the pressure is reduced to 20 mbar.

16. Liquid thickening agent having a skin-care action, prepared by the process according to one or more of Claims 1 to 14, characterised in that the polyglycerol fatty acid ester mixture has (relative to 100 parts by weight of the polyglycerol fatty acid mixture)
(A) a content of
| | |
|---|---|
| 0.1 to 20 % | by weight of monoglycerol fatty acid ester, |
| | |
|---|---|
| 20 to 35 % | by weight of diglycerol fatty acid ester, |
| 30 to 40 % | by weight of triglycerol fatty acid ester and |
| 49.9 to 5 % | by weight of tetra- and higher polyglycerol fatty acid esters, |
the fatty acid component consisting of one or more fatty acids selected from the saturated C₈- to C₁₈-fatty acids, and
(B) a degree of esterification of
| | |
|---|---|
| 20 to 80 % | by weight of fatty acid monoesters and |
| 80 to 20 % | by weight of fatty acid diesters, having a content of 0 to 15 % by weight of tri- and higher esters, |
no or only small amounts (less than 5 % by weight) of free polyglycerol being contained in the polyglycerol fatty acid ester mixture.

17. Liquid thickening agent having a skin-care action according to Claim 16, characterised in that the polyglycerol fatty acid ester mixture has (relative to 100 parts by weight of the polyglycerol fatty acid ester mixture)
(A) a content of
| | |
|---|---|
| 6 to 16 % | by weight of monoglycerol fatty acid ester, |
| 23 to 33 % | by weight of diglycerol fatty acid ester, |
| 31 to 37 % | by weight of triglycerol fatty acid ester and |
| 40 to 14 % | by weight of tetra- and higher polyglycerol fatty acid esters, |
the fatty acid component consisting of one or more fatty acids selected from the saturated C₈- to C₁₈-fatty acids, and
(B) a degree of esterification of
| | |
|---|---|
| 30 to 70 % | by weight of fatty acid monoesters and |
| 70 to 30 % | by weight of fatty acid diesters, having a content of 0.5 to 10 % by weight of tri- and higher esters, |
no or only small amounts (less than 2.5 % by weight) of free polyglycerol being contained in the polyglycerol fatty acid ester mixture.

18. O/W emulsifier having skin-care properties for use in creams or ointments, prepared by the process according to one or more of Claims 1 to 14,
characterised in that the polyglycerol fatty acid ester mixture has (relative to 100 parts by weight of the polyglycerol fatty acid ester mixture)
(A) a content of
| | |
|---|---|
| 0.1 to 20 % | by weight of monoglycerol fatty acid ester, |
| 20 to 35 % | by weight of diglycerol fatty acid ester, |
| 30 to 40 % | by weight of triglycerol fatty acid ester and |
| 49.9 to 5 % | by weight of tetra- and higher polyglycerol fatty acid esters, |
the fatty acid component consisting of one or more fatty acids selected from the saturated C₈- to C₁₈-fatty acids having a content of 0 to 15 % by weight of unsaturated fatty acids, and
(B) a degree of esterification of
| | |
|---|---|
| 20 to 80 % | by weight of fatty acid monoesters and |
| 80 to 20 % | by weight of fatty acid diesters, having a content of 0 to 15 % by weight of tri- and higher esters, |
no or only small amounts (less than 5 % by weight) of free polyglycerol being contained in the polyglycerol fatty acid ester mixture.

19. O/W emulsifier having skin-care properties for use in creams or ointments according to Claim 18, characterised in that the polyglycerol fatty acid ester mixture has (relative to 100 parts by weight of the polyglycerol fatty acid ester mixture)
(A) a content of
| | |
|---|---|
| 6 to 16 % | by weight of monoglycerol fatty acid ester, |
| 23 to 33 % | by weight of diglycerol fatty acid ester, |
| 31 to 37 % | by weight of triglycerol fatty acid ester and |
| 40 to 14 % | by weight of tetra- and higher polyglycerol fatty acid esters, |
the fatty acid component consisting of one or more fatty acids selected from the saturated C₈- to C₁₈-fatty acids having a content of 4 to 10 % by weight of unsaturated fatty acids, and
(B) a degree of esterification of
| | |
|---|---|
| 30 to 70 % | by weight of fatty acid monoesters and |
| 70 to 30 % | by weight of fatty acid diesters having a content of 0.5 to 10 % by weight of tri- and higher esters |
no or only small amounts (less than 2.5 % by weight) of free polyglycerol being contained in the polyglycerol fatty acid ester mixture.

20. Use of the polyglycerol fatty acid ester mixture, prepared by the process according to one or more of Claims 1 to 14, as a solubilising agent, thickening agent, fat-replacing agent and/or emulsifier in cosmetic, pharmaceutical or chemicotechnical preparations.

21. Use of the polyglycerol fatty acid ester mixture according to Claim 20 for the solubilisation and/or emulsification of ethereal oils and perfume oils.

22. Use of the polyglycerol fatty acid ester mixture according to Claim 20 as a skin-care additive and/or detergent, cleaning agent or personal hygiene agent.

23. Use of the polyglycerol fatty acid ester mixture according to Claim 20 as an emulsifier in drilling oils or drilling fluids.

24. Use of the polyglycerol fatty acid ester mixture according to Claim 20 as a wetting and/or dispersing agent in industrial cleaning agents.

25. Use of the polyglycerol fatty acid ester mixture according to Claim 20 as an emulsifier and/or dispersing agent in paint preparations.

## Revendications

1. Procédé de préparation de mélanges d'esters polyglycéroliques d'acides gras par estérification partielle d'un mélange de polyglycérols, formé de mono-,di-,tri-, tétra-glycérols et de polyglycérols supérieurs comportant au moins un acide gras saturé ayant 8 à 18 atomes de carbone, l'acide gras ou le mélange des acides gras présentant une proportion de 0 à 15% en poids d'acides gras insaturés, en présence d'un catalyseur acide, caractérisé en ce qu'on fait réagir un mélange de polyglycérols, formé (par rapport 100% parties en poids de polyglycérols) de :
| | |
|---|---|
| 0,1 à 20% | en poids de monoglycérol |
| 20 à 35% | en poids de diglycérol |
| 30 à 40% | en poids de triglycérol et |
| 5 à 49,9% | en poids de tétraglycérols et de glycérols supérieurs, |
avec l'acide gras ou avec le mélange d'acides gras selon un rapport molaire entre le polyglycérol et l'acide gras ou le mélange des acides gras de 0,3 : 1,5 à 1,5 : 0,5 pour obtenir un mélange d'esters polyglycéroliques d'acides gras formé (par rapport à 100 parties en poids du mélange des esters polyglycéroliques d'acides gras) de :
| | |
|---|---|
| 0,1 à 20% | en poids d'esters monoglycéroliques d'acides gras |
| 20 à 35% | en poids d'esters diglycéroliques d'acides gras |
| 30 à 40% | en poids d'esters triglycéroliques d'acides gras et |
| 49,9 à 5% | en poids d'esters tétrapolyglycéroliques d'acides gras et d'esters polyglycéroliques supérieurs d'acides gras, |
ayant un degré d'estérification de :
| | |
|---|---|
| 20 à 80% | en poids de monoester d'acide gras et |
| 80 à 20% | en poids de diester d'acide gras, avec une proportion de |
| 0 à 15% | en poids de triesters et d'esters supérieur, |
procédé selon lequel on chauffe tout d'abord le mélange réactionnel jusqu'à 140°C et l'on abaisse la pression jusqu'à 600 mbars, puis l'on chauffe le mélange réactionnel, dans un intervalle de la température de 140 à 220°C, par étapes ou en continu, en appliquant un programme de variation de la température, et l'on règle par étapes ou en continu la pression selon un programme de réglage de pression, jusqu'à ce qu'elle soit abaissée à 10 mbars, en chassant en continu, par distillation, l'eau résultant de la réaction et en atteignant un indice d'acide inférieur à 3, on refroidit le mélange polyglycérolique obtenu et éventuellement on en poursuit et termine le traitement et/ou on le purifie.

2. Procédé selon la revendication 1, caractérisé en ce qu'on fait réagir un mélange polyglycérolique formé (par rapport à 100 parties de polyglycérol) de :
| | |
|---|---|
| 6 à 16% | en poids de monoglycérol |
| 23 à 33% | en poids de diglycérol |
| 31 à 37% | en poids de triglycérol, et |
| 14 à 40% | en poids de tétraglycérols et de glycérols supérieurs, |
avec le constituant acide gras pour obtenir un mélange polyglycérolique d'acides gras formé de :
| | |
|---|---|
| 6 à 16% | en poids d'esters monoglycéroliques d'acides gras |
| 23 à 33% | en poids d'esters diglycéroliques d'acides gras |
| 31 à 37% | en poids d'esters d'acides triglycéroliques, et |
| 14 à 40% | en poids d'esters tétraglycéroliques et d'esters polyglycéroliques supérieurs d'acides gras |
ayant un degré d'estérification de :
| | |
|---|---|
| 30 à 70% | en poids de monoester d'acide gras |
| 70 à 30% | en poids de diester d'acide gras, et ayant une proportion de |
| 0,5 à 10% | en poids de triester et d'esters supérieurs. |

3. Procédé selon les revendications 1 ou 2, caractérisé en ce qu'on refroidit à 30 jusqu'à 110°C le mélange des esters polyglycéroliques d'acides gras que l'on obtient ainsi et l'on enlève ensuite, par extraction, les parties polyglycéroliques n'ayant pas réagi, en ajoutant au mélange polyglycérolique d'acides gras un solvant organique chimique ou un mélange de solvants organiques chimiques, puis on extrait avec de l'eau, en au moins une étape d'extraction, et, dans la première étape d'extraction on ajoute en outre une quantité, correspondant à l'indice d'acide du mélange des esters polyglycéroliques d'acides gras et/à la quantité de catalyseur, d'un composé minéral et/ou organique, à réaction basique, et l'on élimine de la phase organique restée après l'extraction, par distillation, avantageusement une distillation sous vide ou une évaporation sous vide, le solvant organique introduit et l'eau résiduelle contenue dans cette phase.

4. Procédé selon une ou plusieurs des revendications 1 à 3, caractérisé en ce que le solvant chimique organique ou le mélange des solvants chimiques organiques possède un pouvoir d'absorption de l'eau inférieur à 30% en poids (par rapport à 100 parties en poids du solvant organique chimique ou du mélange des solvants organiques chimiques) et/ou ce solvant ou ce mélange des solvants forme avec l'eau, lors de la distillation ou en phase gazeuse, un mélange azéotrope.

5. Procédé selon une ou plusieurs des revendications 1 à 4, caractérisé en ce qu'on utilise l'acétate d'éthyle comme solvant chimique organique.

6. Procédé selon une ou plusieurs des revendications 1 à 5, caractérisé en ce qu'on conduit l'extraction en plus de deux étapes.

7. Procédé selon une ou plusieurs des revendications 1 à 6, caractérisé en ce que, pour neutraliser le catalyseur, on ajoute de l'hydroxyde de sodium, dans la première étape d'extraction, au solvant ou au mélange de solvants chimique(s) organique(s) et/ou à la phase aqueuse.

8. Procédé selon une ou plusieurs des revendications 1 à 7, caractérisé en ce que, pour neutraliser le catalyseur on ajoute dans la première phase d'extraction des carbonates alcalins, du carbonate de sodium ou du carbonate de potassium et/ou un échangeur d'ions basique, au solvant ou au mélange des solvants chimique(s) organique(s) et/ou à la phase aqueuse.

9. Procédé selon une ou plusieurs des revendications 1 à 8, caractérisé en ce que on laisse reposer durant plus d'une demi heure le mélange d'esters polyglycéroliques d'acides gras obtenu, avant d'en poursuivre le traitement et/ou de le purifier et l'on en enlève les parties polyglycéroliques éventuellement séparées.

10. Procédé selon une ou plusieurs des revendications 1 à 9, caractérisé en ce que le chauffage du mélange réactionnel dans l'intervalle des températures de 140 à 220°C et l'abaissement de la pression de 600 à 10 mbars ont lieu dans un intervalle de temps de 2 à 6 heures.

11. Procédé selon une ou plusieurs des revendications 1 à 10, caractérisé en ce que le chauffage du mélange réactionnel a lieu dans un intervalle de la température de 140 à 220°C, par étapes en 3 à 6 étapes.

12. Procédé selon une ou plusieurs des revendications 1 à 11, caractérisé en ce que l'abaissement de la pression de 600 à 10 mbars a lieu par étape, en 3 à 6 étapes.

13. Procédé selon une ou plusieurs des revendications 1 à 12, caractérisé en ce qu'on utilise le catalyseur acide, en combinaison avec un composé acide à action réductrice.

14. Procédé selon une ou plusieurs des revendications 1 à 13, caractérisé en ce que l'on fait réagir le mélange réactionnel en présence d'un gaz inerte.

15. Procédé selon la revendication 1 à 14, caractérisé en ce que le rapport molaire du polyglycérol à l'acide gras ou au mélange d'acides gras vaut 0,5:1 à 1,2:1 et pour la réaction, on chauffe tout d'abord le mélange réactionnel à 145°C et l'on abaisse la pression à 500 mbars, puis l'on chauffe le mélange réactionnel à 190°C et l'on abaisse la pression à 20 mbars.

16. Epaississant liquide, doué d'une action de soins de la peau, préparé selon le procédé selon une ou plusieurs des revendications 1 à 14, caractérisé en ce que le mélange des esters polyglycéroliques d'acides gras présente (par rapport à 100 parties en poids du mélange polyglycérolique d'acide gras :
A) Une proportion de :
| | |
|---|---|
| 0,1 à 20% | en poids d'ester monoglycérolique d'acide gras |
| 20 à 35% | en poids d'ester diglycérolique d'acide gras |
| 30 à 40% | en poids d'ester d'acide triglycérolique d'acide gras, et |
| 49,9 à 5% | en poids d'esters tétraglycéroliques et d'esters supérieurs d'acides gras, |
le constituant acide gras consistant en un ou plusieurs acides gras, choisi(s) parmi les acides gras saturés en C₈ à C₁₈, et
B) Présente un degré d'estérification de
| | |
|---|---|
| 20 à 80% | en poids de monoesters d'acides gras, et |
| 80 à 20% | en poids de diesters d'acides gras, ayant une proportion |
| de 0 à 15% | en poids de triesters et d'esters supérieurs, |
des quantités nulles ou faibles seulement (inférieures à 5% en poids) de polyglycérol libre étant contenues dans le mélange d'esters polyglycéroliques d'acides gras.

17. Epaississant liquide ayant une action de soins de la peau, selon la revendication 16, caractérisé en ce que le mélange des esters polyglycéroliques d'acides gras présente, (par rapport à 100 parties en poids du mélange des esters polyglycéroliques d'acides gras) :
A) Une proportion de:
| | |
|---|---|
| 6 à 16% | en poids d'esters monoglycéroliques d'acides gras, |
| 23 à 33% | en poids d'esters diglycéroliques d'acides gras, |
| 31 à 37% | en poids d'esters triglycéroliques d'acides gras et, |
| 40 à 14% | en poids d'esters tétraglycéroliques et d'ester polyglycéroliques supérieurs d'acides gras, |
le constituant acide gras consistant en un ou plusieurs acides gras, choisi(s) parmi les acides gras saturés en C₈ à C₁₈, et,
B) un degré d'estérification de :
| | |
|---|---|
| 30 à 70% | en poids de monoesters d'acides gras, et |
| 70 à 30% | en poids de diesters d'acides gras, ayant une proportion de 0,5 à 10% de triesters et d'esters supérieurs, |
des quantités nulles ou faibles seulement (moins de 2,5% en poids) de polyglycérol libre étant présentes dans le mélange des esters polyglycéroliques d'acides gras.

18. Emulsifiant de type huile dans eau, ayant des propriétés de soins de la peau, pour utilisation dans des crèmes ou des pommades, préparé selon le procédé selon une ou plusieurs des revendications 1 à 14, caractérisé en ce que le mélange des esters polyglycéroliques d'acides gras présente (par rapport à 100 parties en poids du mélange d'esters polyglycéroliques d'acides gras) :
A) Une proportion de:
| | |
|---|---|
| 0,1 à 20% | en poids d'esters monoglycéroliques d'acides gras |
| 20 à 35% | en poids d'esters diglycéroliques d'acides gras |
| 30 à 40% | en poids d'esters triglycéroliques d'acides gras et |
| 49,9 à 5% | en poids d'esters tétraglycéroliques et d'esters polyglycéroliques supérieurs d'acides gras, |
le constituant acide gras consistant en un ou plusieurs acides gras, choisi(s) parmi les acides gras saturés en C₈ à C₁₈, ayant une proportion de 0 à 15% en poids d'acides gras insaturés,
B) Un degré d'estérification de :
| | |
|---|---|
| 20 à 80% | en poids de monoesters d'acides gras et |
| 80 à 20% | en poids de diesters d'acides gras, ayant une proportion de 0 à 15% en poids de triesters et d'esters supérieurs, |
des quantités nulles ou faibles seulement (inférieures à 5% en poids) de polyglycérol libre étant présentes dans le mélange des esters polyglycéroliques d'acides gras.

19. Emulsifiant de type huile dans eau, doué de propriété de soins de la peau (dermatologique) à utiliser dans des crèmes ou des pommades, selon la revendication 18, émulsifiant caractérisé en ce que le mélange des esters polyglycéroliques d'acides gras consiste en (par rapport à 100 parties en poids du mélange des esters polyglycéroliques d'acides gras) :
A) Une proportion de :
| | |
|---|---|
| 6 à 16% | en poids d'esters monoglycéroliques d'acides gras |
| 23 à 33% | en poids d'esters diglycéroliques d'acides gras, |
| 31 à 37% | en poids d'esters triglycéroliques d'acides gras et |
| 40 à 14% | en poids d'esters tétraglycéroliques et d'esters polyglycéroliques supérieurs, |
le constituant acide gras consistant en un ou plusieurs acides gras, choisi(s) parmi les acides gras saturés en C₈ à C₁₈ ayant une proportion de 4 à 10% en poids d'acides insaturés, et
B) Le mélange présente un degré d'estérification de :
| | |
|---|---|
| 30 à 70% | en poids de monoesters d'acides gras et, |
| 70 à 30% | en poids de diesters d'acides gras ayant une proportion |
| de 0,5 à 10% | en poids de triesters et d'esters supérieurs, |
le mélange des esters polyglycéroliques d'acides gras ne contenant pas ou ne contenant que de faibles quantités (inférieures à 2,5% en poids) de polyglycérol libre.

20. Utilisation du mélange d'esters polyglycéroliques d'acides gras, préparé selon le procédé selon une ou plusieurs des revendications 1 à 14, comme solubilisant, épaississant, agent de regraissage et/ou émulsifiant dans des préparations cosmétiques, pharmaceutiques, ou des préparations technico-chimiques.

21. Utilisation du mélange d'esters polyglycéroliques d'acides gras, selon la revendication 20 pour solubiliser et/ou émulsifier des huiles éthérées et des essences de parfums.

22. Utilisation du mélange d'esters polyglycéroliques d'acides gras selon la revendication 20 comme additif pour soins de la peau et/ou comme agent de lavage, de purification ou de nettoyage du corps.

23. Utilisation du mélange d'esters polyglycéroliques d'acides gras selon la revendication 20, comme émulsifiant dans des huiles ou des liquides de perçage.

24. Utilisation du mélange d'esters polyglycéroliques d'acides gras selon la revendication 20, comme agent de mouillage et/ou de dispersion dans des agents techniques de nettoyage.

25. Utilisation du mélange d'esters d'acides gras polyglycéroliques selon la revendication 20 comme émulsifiant et/ou comme dispersant dans des préparations d'encres et de couleurs.
